# FASCICULE DE BREVET EUROPEEN

(11) **EP 3 105 460 B1**
(45) Date de publication et mention de la délivrance du brevet: **02.01.2019**
(21) Numéro de dépôt: 15709239.6
(22) Date de dépôt: 11.02.2015
(51) Int. Cl.: F16B 21/18, F16L 37/084

(54) **CONNECTEUR FLUIDIQUE AVEC BAGUE DE VERROUILLAGE**
FLÜSSIGKEITSKUPPLUNG MIT KLEMMRING
FLUID CONNECTOR WITH LOCKING RING

(30) Priorité: 14.02.2014 FR 1451184
(43) Date de publication de la demande: 21.12.2016
(73) Titulaire: SARTORIUS STEDIM FMT SAS, 13400 Aubagne (FR)
(72) Inventeur: BLAKE, Florian, F-13600 La Ciotat (FR); GIBELIN, Jérémy, F-83330 Le Beausset (FR)
(74) Mandataire: Derambure Conseil
(86) Numéro de dépôt international: PCT/FR2015/050328
(87) Numéro de publication internationale: WO 2015/121581

(56) Documents cités:
- DE-U1- 9 208 976
- DE-U1-202012 104 229
- US-A- 5 074 601

## Description

L'invention concerne les connecteurs fluidiques, en particulier pour les connexions ou raccordements fluidiques permettant de raccorder un connecteur femelle à un connecteur mâle, en vue de raccorder une conduite fluidique à une autre conduite ou à un récipient, dans le domaine des applications biopharmaceutiques.

Plus précisément, on utilise, dans le domaine biopharmaceutique, des conduites ou tubes souples, et des récipients ou contenants, qui servent à transporter et contenir des substances biopharmaceutiques diverses, avec le plus souvent des précautions d'asepsie nécessaires.

Dans le domaine des applications biopharmaceutiques, les fluides sont transportés et stockés à basse pression, c'est-à-dire généralement en dessous de 10 bars et le plus souvent en dessous de 2 bars, voire préférentiellement à une pression proche de la pression atmosphérique.

Dans le cas d'utilisation de certaines substances, il peut être nécessaire de garantir des conditions de stérilité suffisantes après que le raccordement des différents composants (conduites et/ou récipients) ait été effectué. À cette fin, il est commode d'utiliser des raccordements mâle-femelle indémontables après leur accouplement, ce qui permet de garantir qu'un désaccouplement non désiré ne puisse rompre les conditions de stérilité.

Par ailleurs, ce genre de raccordement doit être obtenu par un accouplement simple et rapide. En pratique, on privilégie les solutions où l'accouplement est réalisé par un mouvement de translation axiale sans rotation pour l'insertion du connecteur mâle dans le connecteur femelle.

Selon le but recherché ici, le couplage, une fois obtenu, doit être définitif, ce dernier point excluant de fait toutes les solutions de connecteur déverrouillable utilisées dans le domaine biopharmaceutique.

Il est connu, par exemple du document US 5,074,601 A1, d'utiliser une pièce intermédiaire de verrouillage, interposée radialement entre l'embout mâle et le connecteur femelle, cette pièce intermédiaire ayant la forme d'un collier.

Il est également connu, par exemple du document US5573279, d'agencer des pattes plastiques flexibles à l'intérieur du connecteur femelle, intégralement formées avec ce dernier, lesdites pattes flexibles venant s'arc-bouter derrière un bourrelet de l'embout mâle pour empêcher tout retrait.

Toutefois, les solutions connues présentent divers inconvénients, notamment la complexité des formes des éléments qui concourent au verrouillage de l'embout mâle dans le connecteur femelle, la médiocre compacité radiale et/ou axiale de ces éléments de verrouillage.

Il existe par conséquent le besoin de proposer une amélioration destinée à pallier, au moins en partie, l'inconvénient précité de l'art antérieur connu.

Ci-après, un exposé de l'invention telle que caractérisée dans les revendications.

Selon un premier aspect, l'invention a pour objet un connecteur femelle, pour raccordement fluide, apte et destiné à recevoir un connecteur mâle ayant un rebord annulaire extérieur de retenue, le connecteur femelle comprenant un corps avec un alésage d'axe X, et un logement formant glissière (par exemple une fente) s'étendant généralement dans un plan transversal YZ perpendiculaire à l'axe X, le logement comprenant deux rainures latérales parallèles et le logement étant débouchant à au moins une de ses extrémités selon la direction de la glissière Z, le connecteur femelle comprenant une bague déformable de verrouillage, distincte du corps et rapportée, apte à être insérée dans le logement et à verrouiller le connecteur mâle en position de couplage,
la bague de verrouillage comprenant deux premières portions arquées concaves vu de l'axe X, diamétralement opposées, et deux secondes portions arquées, à courbure inversée, à savoir convexes vu de l'axe X, diamétralement opposées et chacune interposée entre deux premières portions arquées,
les secondes portions arquées étant aptes à fléchir vers l'extérieur lors de l'insertion du connecteur mâle et aptes à venir buter sur le rebord annulaire extérieur de retenue pour empêcher le retrait du connecteur mâle ; moyennant quoi, après verrouillage du connecteur mâle dans le connecteur femelle, on obtient un verrouillage définitif, il n'est plus possible de déverrouiller la connexion par manipulation ou pression sur la bague de verrouillage.

Grâce à ces dispositions, on obtient une bague de verrouillage de très faible encombrement dans le sens axial, et de très bonne compacité dans le sens radial. De plus, la forme de la bague de verrouillage est particulièrement simple, ce qui permet d'obtenir un coût de revient très avantageux.
Il faut remarquer que la connexion est indémontable et qu'on obtiendra une preuve visible si le verrouillage a été violé, par exemple par la matérialisation de la détérioration de la bague de verrouillage.

Selon une réalisation, les premières portions arquées sont en forme d'arc centré sur l'axe X, avec un premier rayon de courbure R1 compris entre 100 % et 200 % du rayon du connecteur mâle, les secondes portions arquées sont en forme d'arc avec un second rayon de courbure R2 compris entre 70 % et 130 % du premier rayon de courbure R1. Moyennant quoi, on assure que les secondes portions arquées ont tendance à se resserrer vers l'axe lorsqu'on appuie sur les premières portions arquées.

Selon une réalisation, le logement et la bague déformable sont dissymétriques par rapport au plan transversal YZ, avec une forme de biseau (ou chanfrein) d'un côté avant de la bague déformable ; de sorte que l'on favorise ainsi, par effet de rampe, l'écartement des secondes portions arquées lors de l'insertion du connecteur mâle, et que l'on ne peut insérer la bague déformable que dans un des deux sens possibles c'est-à-dire avec un détrompage face avant / face arrière.

Selon une réalisation, la bague de verrouillage comprend au moins un ergot saillant en direction axiale, apte à coopérer avec au moins une encoche agencée dans la face avant de la fente formant le logement ; on propose ainsi une solution simple et visuelle permettant un montage adéquat, manuel ou automatisée de la bague dans le bon sens.

Selon une réalisation, la bague déformable comporte des saillies de maintien de la bague dans le logement selon la direction de la glissière Z, avec des formes en creux correspondantes dans les rainures du logement ; moyennant quoi, la bague est maintenue dans le logement après son insertion dans une position substantiellement centrée, avec éventuellement un jeu fonctionnel.

Selon une réalisation, le logement débouche aux deux extrémités selon Z et est symétrique par rapport au plan XY ; moyennant quoi on peut insérer la bague déformable indifféremment depuis chacun des côtés du logement formant glissière.

Selon une réalisation, alternative à la dissymétrie susmentionnée, le logement est symétrique par rapport au plan YZ, de sorte que l'on peut insérer la bague déformable indifféremment dans les deux sens possibles. Moyennant quoi, il est plus facile d'envisager une automatisation du montage.

Selon une réalisation, la bague présente une triple symétrie par rapport aux plans XZ, XY et YZ ; de sorte qu'aucun risque d'erreur n'existe lors du montage de la bague déformable à l'intérieur du logement.

Selon une réalisation, le corps du connecteur femelle est réalisé dans une première matière plastique biocompatible et la bague déformable est réalisée dans une seconde matière plastique; de sorte que l'on peut optimiser les matériaux respectifs du connecteur femelle et de la bague de verrouillage ; en particulier on peut choisir comme seconde matière plastique un matériau aux performances mécaniques optimisées, concernant un compromis rigidité versus élasticité ; En outre, l'optimisation respective des deux matières permet de fabriquer ces composants avec des coûts de revient particulièrement avantageux.

Selon une réalisation, les deuxièmes portions arquées sont inaccessibles lorsque la bague déformable est en position dans le logement formant glissière et les premières portions arquées restent accessibles ; ainsi, on ne peut pas manoeuvrer les secondes portions arquées et une pression radiale exercée vers l'intérieur sur les premières portions arquées tend à accentuer la courbure des deuxièmes portions arquées et donc tend à renforcer le verrouillage ; de sorte qu'il est impossible de déverrouiller la connexion, même involontairement ou en cas de chute ou de choc.

Selon une réalisation, lorsque la bague déformable est en position dans le connecteur femelle, les premières portions arquées ne dépassent pas de la surface du corps du connecteur femelle, et sont de préférence affleurantes ; de sorte que l'on peut exercer une pression en direction de l'axe sur les premières portions arquées mais qu'on ne peut pas manuellement exercer une traction en direction opposée de l'axe ; moyennant quoi cette disposition concourt à garantir le caractère non déverrouillable de du raccordement.

Selon un deuxième aspect, l'invention a pour objet une bague déformable moulée en matière plastique convenant pour être utilisée comme une bague de verrouillage dans un connecteur femelle tel que décrit précédemment, la bague déformable comprenant deux premières portions arquées concaves vu de l'axe X, diamétralement opposées, et deux secondes portions arquées, à courbure inversée, à savoir convexes vu de l'axe X, diamétralement opposées et chacune interposée entre deux premières portions arquées.

On décrit maintenant brièvement les figures des dessins.
La figure 1 est une vue en perspective du dispositif de connexion conforme à la présente invention.
La figure 2 est une vue en coupe axiale du dispositif de connexion de la figure 1, en position accouplée, selon la ligne de coupe II-II visible sur la figure 5.
La figure 3 est une vue en perspective de la bague de verrouillage.
La figure 4 est vue en perspective d'une coupe transversale du dispositif de connexion.
La figure 5 montre une coupe transversale du dispositif de connexion.
La figure 6 montre une vue de dessus du connecteur femelle sans la bague de verrouillage.
Les figures 7A et 7B représentent deux variantes de profil de l'embout mâle avec une bague de verrouillage respectivement symétrique et dissymétrique par rapport au plan YZ.
Les figures 8A, 8B et 8C illustrent une autre variante avec un détrompage de position affectant la bague de verrouillage et le logement dans le connecteur femelle.

Ci-après un exposé détaillé de plusieurs modes de réalisation de l'invention assorti d'exemples et de référence aux dessins.

Dans l'exemple illustré, il s'agit de raccorder un connecteur femelle **1** à un connecteur mâle **2**, par un mouvement d'accouplement au cours duquel on insère le connecteur mâle dans le connecteur femelle selon une direction axiale **X**.

Pour chacun de ces connecteurs, dans le présent document, le terme **'avant'** désigne la partie la plus proche du connecteur opposé complémentaire, et le terme **'arrière'** désigne la partie la plus éloignée du connecteur opposé.

Le connecteur femelle **1** comprend dans sa portion arrière un embout tubulaire **17** destiné à recevoir un tube souple (non représenté) et dans sa portion avant une interface de couplage avec le connecteur mâle **2**, ladite interface de couplage sera décrite plus bas.
De façon similaire, le connecteur mâle **2** comprend dans sa portion arrière un embout tubulaire **27** destiné à recevoir un tube souple et dans sa portion avant une interface de couplage avec le connecteur femelle, dont il sera question plus loin.
Il faut remarquer ici que le connecteur femelle et/ou le connecteur **mâle** pourraient présenter dans leurs portions arrière, à la place d'un embout de réception de tube tel que représenté, une collerette de raccord à un récipient tel que par exemple une poche souple ou rigide ou encore un filtre.

Chacun des embouts tubulaires **17,27** est de révolution autour de l'axe X, et ils comprennent chacun un ou plusieurs crans **19,29** de rétention extérieurs pour accrocher le tube souple, et une collerette d'arrêt **18,28** sur laquelle peut venir buter le tube souple lors de son insertion sur l'embout. Comme connu en soi, on peut ajouter un collier de serrage autour du tube souple et de l'embout.

Le connecteur mâle **2** comprend un passage axial **26** de révolution autour de l'axe X, qui permet le passage du fluide transporté au travers du raccordement fluidique.
En partant de sa portion extrême avant, le connecteur mâle comprend tout d'abord un chanfrein d'entrée **25**, puis une gorge annulaire **21** destinée à recevoir un joint torique d'étanchéité **9** classique, puis une première portion intermédiaire **20** équipée de nervures longitudinales **73**, puis une partie tronconique **23** qui s'évase vers l'extérieur et vers l'arrière du connecteur mâle.
Toujours en se dirigeant vers l'arrière du connecteur, la partie tronconique **23** se prolonge par un rebord annulaire **24**, qui forme un épaulement destiné à porter sur une autre surface en contrepartie et à empêcher le retrait du connecteur mâle comme il sera vu plus loin.
En arrière du rebord annulaire anti retrait **24**, le connecteur mâle comprend une seconde portion intermédiaire **22** avec un anneau **72** équipé de quatre petits renforts **74**, ledit anneau avec ces renforts servant de moyens de préhension pour pousser le connecteur mâle en direction du connecteur femelle et servant également de butée axiale pour le mouvement d'insertion.
En arrière de l'anneau **72**, on trouve la collerette **28** et l'embout tubulaire proprement dit **27** déjà commentés.

Le connecteur femelle **1** comprend un passage axial **15** de révolution autour de l'axe X, qui permet le passage du fluide transporté au travers du raccordement fluidique.
En partant de sa portion extrême arrière, le connecteur femelle 1 comprend l'embout tubulaire **17** déjà commenté, puis un corps **10** principal destiné à recevoir la portion avant du connecteur mâle, avec un alésage de diamètre légèrement supérieur au diamètre extérieur de la portion avant de l'embout mâle **2**, de manière à pouvoir permettre l'insertion de la partie avant du connecteur mâle et de manière à pouvoir exercer une pression radiale sur le joint torique **9** pour assurer la fonction étanchéité. En vis-à-vis de la partie tronconique **23**, le corps **10** présente une dimension intérieure qui va en s'accroissant jusqu'à l'emplacement d'un logement **4** pour la bague de verrouillage **3** dont il va être question plus loin.
En effet, dans la portion avant du connecteur femelle est agencé un logement formant glissière **4** s'étendant dans un plan transversal **YZ** perpendiculaire à l'axe **X**. En l'occurrence, le logement formant glissière se présente ici comme une fente transversale. De plus, dans l'exemple illustré, cette fente débouche des deux côtés **40,46** selon la direction transversale **Z** qui permet l'insertion d'une bague de verrouillage.
Dans l'exemple illustré, le logement 4 comprend une première rainure **41** et une seconde de rainure **42** lesquelles se font face et son disposées symétriquement par rapport au plan **XZ**.
Enfin, le corps **10** du connecteur femelle comprend un anneau frontal **11** qui forme l'extrémité avant et sur lequel viendra buter en fin de mouvement d'insertion l'anneau **72** du connecteur mâle. Le corps **10** du connecteur femelle comporte aussi en arrière du logement un anneau arrière **12**, et par conséquent le logement 4 est interposées entre l'anneau frontal **11** et l'anneau arrière **12**.

Il faut remarquer ici qu'il n'est pas strictement nécessaire que la fente 4 soit débouchante des deux côtés, en effet en variante non représentée, un des côtés pourrait être au moins partiellement fermé ce qui formerait un fond pour le logement 4 tandis que le côté opposé constituerait l'embouchure par laquelle il faut insérer la bague de verrouillage 3.

La bague de verrouillage **3** déjà évoquée est destinée à être insérée dans le logement **4** susmentionné du corps du connecteur femelle. La bague de verrouillage **3** est formée comme un anneau fermé déformable, c'est-à-dire notamment qu'elle peut être déformée radialement vers l'extérieur lors du mouvement d'insertion du connecteur mâle dans le connecteur femelle.

Plus précisément, la bague de verrouillage comprend deux premières portions arquées **31**, concaves vu de l'axe **X**, diamétralement opposées, et deux secondes portions arquées **32**, à courbure inversée, à savoir convexes vu de l'axe X, diamétralement opposées et chacune interposée entre deux premières portions arquées. Autrement dit, si on parcourt la bague dans la direction circonférentielle, on trouve une première portion arquée, puis une deuxième portion arquée à courbure inversée, puis une autre première portion arquée et enfin une autre deuxième portion arquée à courbure inversée. Il faut remarquer que la section de chacune des portions arquées reste relativement constante, et que de plus la section de l'anneau n'évolue pas substantiellement lorsqu'on passe d'une première section arquée à une deuxième section arquée ou vice versa.

Les premières portions arquées **31** sont concaves vu de l'axe **X**, c'est-à-dire que leur courbure est dirigée vers l'intérieur de la bague alors que secondes portions arquées **32** sont convexes vu de l'axe X c'est-à-dire que leur courbure est dirigée vers l'extérieur de la bague.

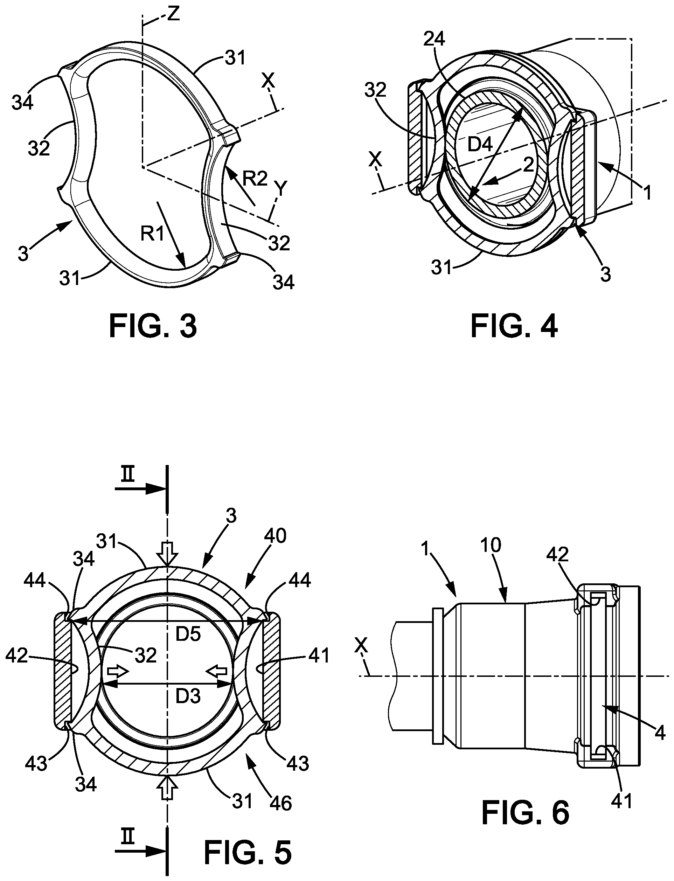

Avantageusement, les premières portions arquées **31** sont ici en forme d'arc centré sur l'axe **X**, avec un premier rayon de courbure **R1** (Fig.3) compris entre 100 % et 200 % du rayon **R0** du connecteur mâle, les secondes portions arquées **32** sont en forme d'arc avec un second rayon de courbure **R2** compris entre 70 % et 130 % du premier rayon de courbure **R1**.

Les secondes portions arquées **32** sont aptes à fléchir vers l'extérieur lors de l'insertion du connecteur mâle en particulier lorsque la forme tronconique **23** vient s'appuyer sur la bague. Après insertion complète du connecteur mâle, le rebord annulaire extérieur **24** a dépassé la bague, et la bague est rappelée vers sa position de repos et vient alors buter sur le rebord annulaire extérieur **24** de retenue pour empêcher le retrait du connecteur mâle.
Lorsque la bague de verrouillage **3** est au repos, la distance **D3** (Fig 4 et 5) entre les deux secondes portions arquées est légèrement inférieure au diamètre **D4** du connecteur mâle de la deuxième portion intermédiaire **22** en arrière du rebord annulaire **24**.

Il faut remarquer que les deuxièmes portions arquées **32** sont inaccessibles depuis l'extérieur lorsque la bague déformable est en position dans le logement **4** et seules les premières portions arquées **31** restent accessibles dans une certaine mesure ; mais comme ceci est illustré à la figure 5, une pression radiale vers l'intérieur tend à accentuer la courbure des deuxièmes portions arquées et donc tend à renforcer le verrouillage ; de sorte qu'il est impossible de déverrouiller la connexion, même involontairement ou en cas de chute ou de choc du connecteur contre un objet.

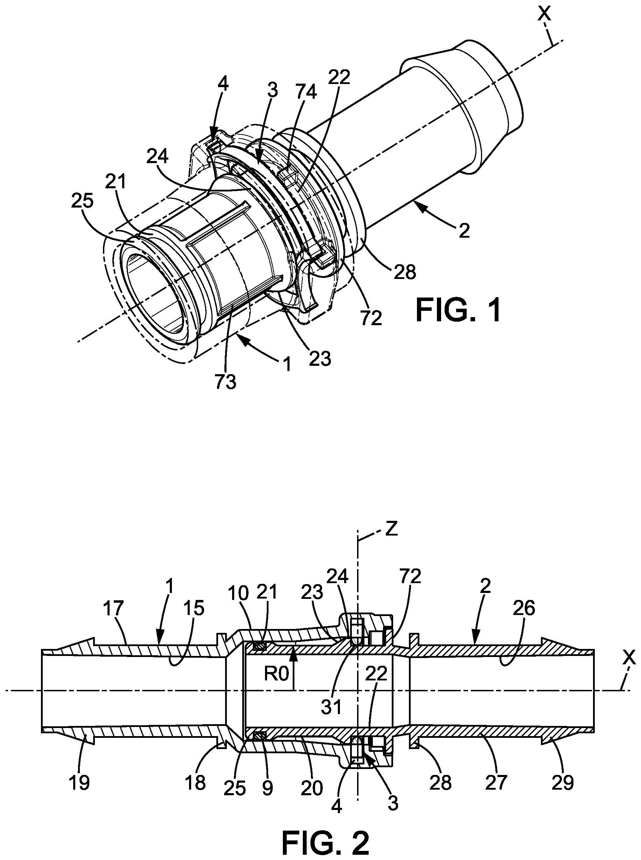

Avantageusement, lorsque la bague déformable **3** est en position dans le logement, les premières portions arquées **31** ne dépassent pas de la surface du corps du connecteur femelle, et par conséquent il n'est pas possible de les attraper avec les doigts pour essayer de déverrouiller la connexion. On remarque que sur l'exemple illustré aux figures 1 et 4, les premières portions arquées **31** affleurent à la surface à la fois de l'anneau arrière **12** et de l'anneau avant **11**. Il n'y a pas non plus de jeu dans le sens axial qui permettrait d'insérer un objet pour tenter d'écarter les premières portions radiales pour essayer de violer le verrouillage de la connexion. Par conséquent, l'inaccessibilité des secondes portions arquées **32** tout comme l'impossibilité d'exercer une traction sur les premières portions arquées **31** conduisent au résultat qu'il n'est pas possible par une manoeuvre d'un utilisateur de pouvoir déverrouiller la connexion. Seule une détérioration substantielle de la bague de verrouillage **3** ou du corps **10** du connecteur femelle peut conduire à une telle éventualité ; mais alors il y aurait une preuve visible sur les pièces.
Par ailleurs on peut remarquer la compacité remarquable dans la direction radiale de cette solution, en effet le diamètre hors tout extérieur de la bague de verrouillage n'est pas supérieur à 125% du diamètre **D4**, c'est-à-dire n'est pas supérieur à 250% du rayon **R0** de la portion avant du connecteur mâle. On obtient également un faible encombrement de la solution dans la direction axiale car l'épaisseur de la bague déformable **3** selon **X** est plusieurs fois inférieure au diamètre utile du passage axial du raccordement.

On peut noter la disposition symétrique avantageuse de la bague déformable **3**, elle est en effet symétrique par rapport au plan **XZ** et au plan **XY**.

De même, il faut noter que les rainures **41** et **42** pourraient être partiellement ajourées, il n'est pas nécessaire qu'elles soient complètement fermées, il suffit qu'elles rendent difficile l'accès aux deuxièmes portions arquées **32**.

Préalablement à l'insertion du connecteur mâle, la bague de verrouillage **3** peut être maintenue dans une position dite d'attente à l'intérieur du logement par différents moyens. Dans l'exemple illustré, la bague de verrouillage est maintenue dans le logement par des formes en saillie **34**, agencées substantiellement au niveau des jonctions entre les premières portions arquées et les secondes portions arquées, ce qui donne quatre formes en saillie **34**, que l'on pourrait aussi appeler crochets, becs ou griffes. Lesdites formes en saillie **34** s'étendent vers l'extérieur généralement selon la direction **Y**. Il est prévu des formes en creux **43,44** correspondantes en vis-à-vis dans les rainures **41, 42**, ces formes en creux étant destinées à recevoir les formes en saillie de la bague de verrouillage lorsque celle-ci arrive dans sa position sensiblement centrée sur l'axe **X**.

Avantageusement, on utilise les propriétés de flexibilité de la bague non seulement pour l'accouplement du connecteur mâle dans le connecteur femelle, mais aussi pour l'insertion préalable de la bague de verrouillage 3 dans la fente 4 le long de la direction Z. Plus précisément, la distance selon Y entre les extrémités des formes en saillie 34 est légèrement supérieure à la distance D5 qui sépare le fond des rainures 41, 42. Ainsi, pendant l'insertion, les formes en saillie **34** sont légèrement comprimées vers l'intérieur afin de glisser le long de l'intérieur de chacune des rainures 41,42, et de revenir vers la position de repos dès lors que les formes en saillie **34** sont en face des formes en creux **43,44** susmentionnées. Un positionnement précis de la bague n'est pas forcément requis, on peut avoir un jeu fonctionnel pour autant que la bague soit à peu près centrée sur l'axe **X**.

Le connecteur femelle **1** et le connecteur mâle **2** peuvent être obtenus par moulage d'une première matière plastique, en particulier des matières plastiques bio compatibles avec les diverses substances biopharmaceutiques visées, par exemple polypropylène, du polyéthylène, du polycarbonate, du polysulfone, polychlorure de vinylidène, polyethylenimine. On choisira de préférence le polyéthylène ou le polypropylène.
La bague de verrouillage déformable **3** peut être elle aussi réalisée dans la même matière par exemple polybutylène téréphtalate, polypropylène, polyéthylène, polycarbonate, polyoxyméthylène; alternativement, et avantageusement dans certains cas, on pourra choisir pour la bague déformable 3 une seconde matière plastique différente des connecteurs, typiquement du polybutylène téréphtalate ou du polycarbonate, avec des propriétés mécaniques et élastiques plus ciblées, car en effet la bague de verrouillage n'est jamais en contact avec les substances transportées dans la conduite objet du raccordement. On pourra optimiser le compromis de la rigidité par rapport à l'élasticité du second matériau. On pourra aussi prendre en compte l'évolution des qualités du matériau en fonction du vieillissement naturel ou provoqué par la lumière ou d'autres rayonnements, ou d'autres agressions physico-chimiques.

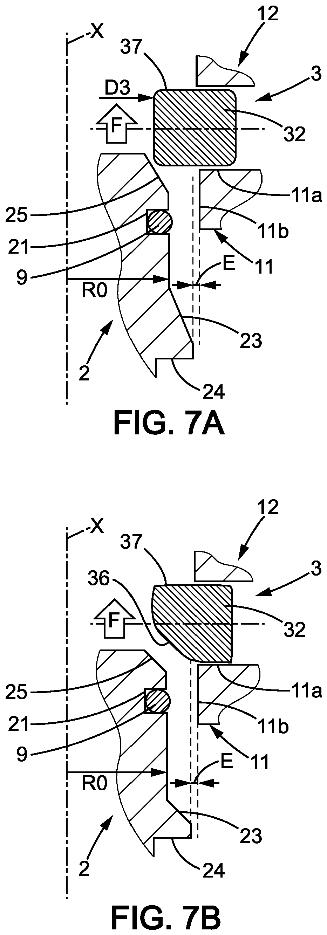

Comme illustré à la figure 7A, dans le cas où le connecteur mâle 2 présente des rampes d'entrée **25,23** à faible pente, on peut utiliser une section de deuxième portion arquée **32** symétrique par rapport au plan **YZ**. Dans ce cas de figure, on peut former la bague de verrouillage avec une triple symétrie, à savoir une triple symétrie par rapport aux plans **XZ**, **XY** et **YZ**, de sorte qu'aucun risque d'erreur n'existe lors du montage de la bague déformable à l'intérieur du logement 4. De plus cette disposition de triple symétrie peut faciliter l'automatisation de l'insertion de la bague dans un connecteur femelle, les bagues de verrouillage pouvant être acheminées à la queue leu leu au moyen d'un pot vibrant relativement simple, vers un robot manipulateur.

Comme illustré à la figure 7B, pour faciliter la déflection de la bague déformable au moment de l'insertion, par exemple dans le cas où les rampes d'entrée **25,23** sont plus prononcées, on prévoit une face biseautée **36** sur la face avant de la bague qui permet de diminuer les efforts d'insertion **F** et de faciliter l'écartement des deuxièmes portions arquées 32 de la bague de verrouillage. En revanche, sur le côté opposé de la section de la bague (face arrière **37**), on conserve un profil droit afin que le rebord extérieur **24** du connecteur mâle porte bien à plat sur la face arrière **37** de la bague. Ainsi, une traction sur l'embout mâle après accouplement n'aura pas tendance à écarter les secondes portions arquées 32.

Il faut noter que cette portée « à plat » prévaut aussi dans le cas précédent de la figure 7A.

On remarque un jeu fonctionnel **E** entre l'embout mâle et le diamètre intérieur **11b** qui permet de garantir que l'embout mâle y compris l'épaulement **24** passe au-delà de l'anneau avant **11** de la fente du connecteur femelle 1.

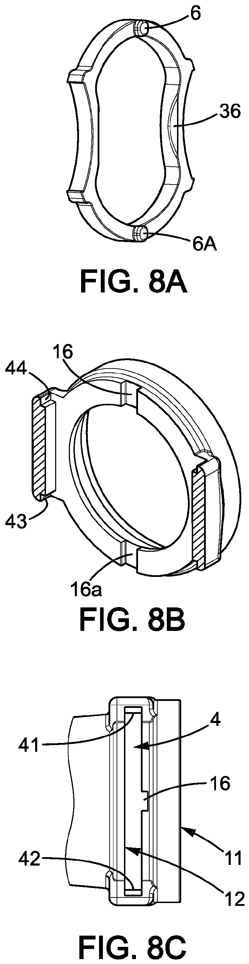

Selon une caractéristique optionnelle, illustrée aux figures 8A, 8B, 8C, on peut prévoir une fonction de détrompage pour garantir le sens de montage de la bague de déverrouillage à l'intérieur du logement **4**. En particulier, lorsque qu'on utilise un profil de section de bague dissymétrique (Fig 7B) dans les deuxièmes portions arquées **32**, il faut garantir que la portion biseautée **36** est placée du côté de l'insertion du connecteur mâle.
À cette fin, deux ergots **6,6A** sont agencés dans les parties médianes des premières portions arquées **31** et s'étendent (venus de matière) en direction de l'axe **X**. Deux encoches **16,16A** formées de façon complémentaire aux ergots **6,6A** susmentionnés, sont ménagées dans le plan médian **XZ** de la fente (sur la paroi avant de la fente), une en haut et une en bas. Ainsi, si on ne peut pas insérer la bague déformable à l'envers avec la partie biseautée dirigée du mauvais côté, car l'ergot 6 buterait sur l'anneau arrière **12** dépourvu d'encoche.

Il faut remarquer ici que si le logement ne permettait l'insertion de la bague **3** que d'un seul côté débouchant, par exemple par le haut, une seule encoche **16** serait nécessaire.

On remarque que dans l'exemple illustré, le connecteur mâle peut tourner à l'intérieur du connecteur femelle autour de **X**, il n'y a pas de position en orientation particulière pour insérer le connecteur mâle dans le connecteur femelle.

Toutefois, il n'est pas exclu de prévoir un dispositif anti rotation qui peut par exemple être agencé entre les petits renforts **74** de l'anneau **72** du connecteur mâle et l'anneau frontal **11** du connecteur femelle.

Avantageusement selon l'invention, la forme particulière de la bague de verrouillage avec notamment les secondes portions arquées, présente un avantage concernant la robustesse du verrouillage ; en effet en cas de choc non intentionnel, ou en cas de tentative de manoeuvre frauduleuse de la connexion, les secondes portions arquées ont tendance à se déplacer vers l'intérieur et par conséquent renforcer l'effet de verrouillage au lieu de le diminuer. Par conséquent, seule une détérioration physique de la bague de verrouillage (qui serait alors visible) pourrait conduire à un déverrouillage de la connexion.

## Revendications

1. Connecteur femelle (1), pour raccordement fluidique, apte et destiné à recevoir un connecteur mâle (2) ayant un rebord annulaire (24) extérieur de retenue, le connecteur femelle comprenant un corps (10) avec un alésage d'axe X, et un logement formant glissière (4) s'étendant généralement dans un plan transversal YZ perpendiculaire à l'axe X, le logement (4) comprenant deux rainures latérales (41,42) parallèles et le logement étant débouchant à au moins une de ses extrémités selon la direction de la glissière Z,
le connecteur femelle comprenant une bague déformable de verrouillage (3) distincte du corps, apte à être insérée dans le logement et apte à verrouiller le connecteur mâle en position de couplage,
la bague de verrouillage comprenant deux premières portions arquées (31), concaves vu de l'axe X, diamétralement opposées, et deux secondes portions arquées (32), à courbure inversée, à savoir convexes vu de l'axe X, diamétralement opposées et chacune interposée entre deux premières portions arquées,
les secondes portions arquées étant aptes à fléchir vers l'extérieur lors de l'insertion du connecteur mâle et aptes à venir buter sur le rebord annulaire extérieur de retenue pour empêcher le retrait du connecteur mâle, moyennant quoi, après verrouillage du connecteur mâle dans le connecteur femelle, on obtient un verrouillage définitif.

2. Connecteur selon la revendication 1, dans lequel les premières portions arquées (31) sont en forme d'arc centré sur l'axe X, avec un premier rayon de courbure (R1) compris entre 100 % et 200 % du rayon (R0) extérieur de la portion avant du connecteur mâle, les secondes portions arquées (32) sont en forme d'arc avec un second rayon de courbure (R2) compris entre 70 % et 130 % du premier rayon de courbure (R1).

3. Connecteur selon l'une des revendications 1-2, dans lequel le logement (4) et la bague déformable (3) sont dissymétriques par rapport au plan transversal YZ, avec une forme de biseau (36) d'un côté avant de la bague déformable.

4. Connecteur selon la revendication 3, dans lequel la bague de verrouillage comprend au moins un ergot (6,6A) saillant en direction axiale, apte à coopérer avec au moins une encoche (16) agencée dans la face avant de la fente formant le logement.

5. Connecteur selon l'une des revendications 1-4, dans lequel la bague déformable comporte des saillies (34) de maintien de la bague dans le logement selon la direction de la glissière Z, avec des formes en creux (43,44) correspondantes dans les rainures du logement.

6. Connecteur selon l'une des revendications 1-5, dans lequel le logement débouche aux deux extrémités selon Z et est symétrique par rapport au plan XY, la bague déformable pouvant être insérée indifféremment depuis chacun des côtés du logement formant glissière.

7. Connecteur selon la revendication 6, dans lequel le logement est symétrique par rapport au plan YZ de sorte que l'on peut insérer la bague déformable indifféremment dans les deux sens possibles.

8. Connecteur selon la revendication 7, dans lequel la bague déformable (3) présente une triple symétrie, par rapport aux plans XZ, XY et YZ.

9. Connecteur selon l'une des revendications 1-8, dans lequel le corps du connecteur femelle (1) est réalisé dans une première matière plastique biocompatible et la bague déformable (3) est réalisée dans une seconde matière plastique.

10. Connecteur selon l'une des revendications 1-9, dans lequel les deuxièmes portions arquées (32) sont inaccessibles lorsque la bague déformable est en position dans le logement formant glissière et seules les premières portions arquées (31) restent accessibles.

11. Connecteur selon l'une des revendications 1-9, dans lequel lorsque la bague déformable est en position dans le connecteur femelle, les premières portions arquées (31) ne dépassent pas de la surface du corps (10) du connecteur femelle, et sont de préférence affleurantes.

12. Bague déformable (3) moulée en matière plastique, convenant pour être utilisée comme une bague de verrouillage dans un connecteur femelle (1) selon l'une des revendications précédentes, la bague déformable comprenant deux premières portions arquées (31) concaves vu de l'axe, diamétralement opposées, et deux secondes portions arquées (32), à courbure inversée, à savoir convexes vu de l'axe, diamétralement opposées et chacune interposée entre deux premières portions arquées,, la bague comprenant des formes en saillie (34) s'étendant vers l'extérieur, agencées aux jonctions entre les premières portions arquées (31) et les secondes portions arquées (32), pour le maintien de la bague dans un logement.

## Patentansprüche

1. Weibliches Verbindungselement (1) für eine Fluidverbindung, welches dazu eingerichtet und vorgesehen ist, ein männliches Verbindungselement (2) aufzunehmen, welches einen ringförmigen äußeren Halterand (24) aufweist, wobei das weibliche Verbindungselement einen Körper (10) mit einer Bohrungsachse X und eine eine Gleitführung (4) bildende Aufnahme umfasst, welche sich im Wesentlichen in einer transversalen Ebene YZ senkrecht zu der Achse X erstreckt, wobei die Aufnahme (4) zwei parallele laterale Nuten (41, 42) umfasst und sich die Aufnahme an wenigstens einem ihrer Enden entlang der Gleitrichtung Z öffnet,
wobei das weibliche Verbindungselement einen von dem Körper separaten deformierbaren Verriegelungsring (3) umfasst, welcher dazu eingerichtet ist, in die Aufnahme eingesetzt zu sein und dazu eingerichtet ist, das männliche Verbindungselement in Kopplungsposition zu verriegeln,
wobei der Verriegelungsring zwei diametral entgegengesetzte, aus der Achse X betrachtet konkave erste Bogenabschnitte (31) und zwei invers gebogene, das heißt aus der Achse X betrachtet konvexe zweite Bogenabschnitte (32) umfasst, welche diametral entgegengesetzt sind und jeweils zwischen zwei ersten Bogenabschnitten eingesetzt sind,
wobei die zweiten Bogenabschnitte dazu eingerichtet sind, sich während des Einsetzens des männlichen Verbindungselements nach außen zu biegen und dazu eingerichtet sind, an dem ringförmigen äußeren Halterand zur Anlage zu kommen, um das Entfernen des männlichen Halteelements zu verhindern, wodurch nach dem Verriegeln des männlichen Verbindungselements in dem weiblichen Verbindungselement eine definitive Verriegelung erhalten wird.

2. Verbindungselement nach Anspruch 1, wobei die ersten Bogenabschnitte (31) in Form eines Bogens mit Mitte auf der Achse X mit einem ersten Krümmungsradius (R1) sind, welcher zwischen 100% und 200% des äußeren Radius (R0) des vorderen Abschnitts des männlichen Verbindungselements beträgt, wobei die zweiten Bogenabschnitte (32) in Form eines Bogens mit einem zweiten Krümmungsradius (R2) sind, welcher zwischen 70% und 130% des ersten Krümmungsradius (R1) beträgt.

3. Verbindungselement nach einem der Ansprüche 1-2, wobei die Aufnahme (4) und der deformierbare Ring (3) asymmetrisch bezüglich der transversalen Ebene YZ sind, mit Form einer Abschrägung (36) einer vorderen Seite des deformierbaren Rings.

4. Verbindungselement nach Anspruch 3, wobei der Verriegelungsring wenigstens einen Zapfen (6, 6A) umfasst, welcher in axialer Richtung vorsteht, dazu eingerichtet, mit wenigstens einer Kerbe (16) zusammenzuwirken, welche an der vorderen Fläche des Spalts angeordnet ist, welcher die Aufnahme bildet.

5. Verbindungselement nach einem der Ansprüche 1-4, wobei der deformierbare Ring Vorsprünge (34) zum Halten des Rings in der Aufnahme entlang der Gleitrichtung Z umfasst, mit entsprechenden Hohlformen (43, 44) in den Nuten der Aufnahme.

6. Verbindungselement nach einem der Ansprüche 1-5, wobei die Aufnahme an zwei Enden entlang Z mündet und bezüglich der Ebene XY symmetrisch ist, wobei der deformierbare Ring gleichermaßen von jeder der Seiten der die Gleitführung bildenden Aufnahme eingeführt werden kann.

7. Verbindungselement nach Anspruch 6, wobei die Aufnahme bezüglich der Ebene YZ derart symmetrisch ist, dass der deformierbare Ring gleichermaßen in den beiden möglichen Orientierungen eingeführt werden kann.

8. Verbindungselement nach Anspruch 7, wobei der deformierbare Ring (3) eine dreifache Symmetrie aufweist, bezüglich den Ebenen XZ, XY und YZ.

9. Verbindungselement nach einem der Ansprüche 1-8, wobei der Körper des weiblichen Verbindungselements (1) aus einem ersten, biokompatiblen Kunststoffmaterial hergestellt ist und der deformierbare Ring (3) aus einem zweiten Kunststoffmaterial hergestellt ist.

10. Verbindungselement nach einem der Ansprüche 1-9, wobei die zweiten Bogenabschnitte (32) unzugänglich sind, wenn der deformierbare Ring in Position in der die Gleitführung bildenden Aufnahme ist und lediglich die ersten Bogenabschnitte (31) zugänglich bleiben.

11. Verbindungselement nach einem der Ansprüche 1-9, wobei während der verformbare Ring in Position in dem weiblichen Verbindungselement ist, die ersten Bogenabschnitte (31) die Fläche des Körpers (10) des weiblichen Verbindungselements nicht durchtreten und vorzugsweise bündig sind.

12. Deformierbarer Ring (3), welcher aus Kunststoff gegossen ist, dazu geeignet, als Verriegelungsring in einem weiblichen Verbindungselement (1) nach einem der vorhergehenden Ansprüche verwendet zu werden, wobei der deformierbare Ring zwei diametral entgegengesetzte, aus der Achse betrachtet konkave erste Bogenabschnitte (31) und zwei invers gebogene, das heißt aus der Achse betrachtet konvexe zweite Bogenabschnitte (32) umfasst, welche diametral entgegengesetzt sind und jeweils zwischen zwei ersten Bogenabschnitten eingesetzt sind, wobei der Ring vorspringende Formen (34) umfasst, welche sich nach außen erstrecken, angeordnet an Verbindungsstellen zwischen den ersten Bogenabschnitten (31) und den zweiten Bogenabschnitten (32), für das Halten des Rings in einer Aufnahme.

## Claims

1. Female connector (1), for fluid connection, adapted for receiving a male connector (2) having a retaining external annular rim (24), the female connector comprising a body (10) with a bore of axis X, and a housing forming a slide (4) extending generally within a transverse plane YZ perpendicular to axis X, the housing 4 comprising two parallel lateral grooves (41,42) and the housing being open at least at one of its ends in the direction Z of the slide,
the female connector comprising a deformable locking ring (3) distinct from the body, adapted to be inserted into the housing and to lock the male connector in the coupling position,
the locking ring comprising two diametrically opposed first arched portions (31), concave as viewed from the axis X, and two diametrically opposed second arched portions (32) of inverse curvature, meaning convex as viewed from the axis X, each of said second arched portions being interposed between two first arched portions,
the second arched portions being adapted to flex outward during insertion of the male connector and to abut against the retaining external annular rim to prevent withdrawal of the male connector, whereby, after locking the male connector in the female connector, a definitive locking is obtained.

2. Connector according to claim 1, wherein the first arched portions (31) each form an arc centered on axis X, with a first radius of curvature (R1) of between 100% and 200% of the external radius (R0) of the front portion of the male connector, the second arched portions (32) each form an arc with a second radius of curvature (R2) of between 70% and 130% of the first radius of curvature (R1).

3. Connector according to one of claims 1-2, wherein the housing (4) and the deformable ring (3) are asymmetrical with respect to the transverse plane YZ, with the front side of the deformable ring having a beveled shape (36).

4. Connector according to claim 3, wherein the locking ring comprises at least one pin (6,6A) projecting in the axial direction, able to engage with at least one notch (16) arranged in the front face of the slot forming the housing.

5. Connector according to one of claims 1-4, wherein the deformable ring comprises projections (34) for retaining the ring in the housing along the direction Z of the slide, with corresponding recesses (43,44) formed in the grooves of the housing.

6. Connector according to one of claims 1-5, wherein the housing is open at both ends along Z and is symmetrical with respect to the XY plane, it being possible to insert the deformable ring from either side of the housing forming the slide.

7. Connector according to claim 6, wherein the housing is symmetrical with respect to the YZ plane, such that the deformable ring can be inserted in either of the two possible directions.

8. Connector according to claim 7, wherein the deformable ring has triple symmetry, with respect to planes XZ, XY, and YZ.

9. Connector according to one of claims 1-8, wherein the body of the female connector (1) is made of a first biocompatible plastic material and the deformable ring (3) is made of a second plastic material.

10. Connector according to one of claims 1-9, wherein the second arched portions (32) are inaccessible when the deformable ring is in position in the housing forming the slide and only the first arched portions (31) remain accessible.

11. Connector according to one of claims 1-9, wherein, when the deformable ring is in position in the female connector, the first arched portions (31) do not protrude from the surface of the body (10) of the female connector, and are preferably flush with it.

12. Deformable **ring** (3) of molded plastic, suitable for use as a locking ring in a female connector (1) according to one of the preceding claims, the deformable ring comprising two diametrically opposed first arched portions (31), concave as viewed from the axis, and two diametrically opposed second arched portions (32) of inverse curvature, meaning convex as viewed from the axis, each of said second arched portions being interposed between two first arched portions, the ring comprising outwardly extending projections (34) arranged at the joining between the first arched portions (31) and the second arched portions (32), for retaining the ring in a housing.
